# FASCICULE DE BREVET EUROPEEN

(11) **EP 3 710 011 B1**
(45) Date de publication et mention de la délivrance du brevet: **29.11.2023**
(21) Numéro de dépôt: 18803636.2
(22) Date de dépôt: 13.11.2018
(51) Int. Cl.: A61K 31/56, A61P 1/02

(54) **TRITERPÈNES PENTACYCLIQUES DANS LE TRAITEMENT D'UNE PATHOLOGIE BUCCO-DENTAIRE**
PENTACYCLISCHE TRITERPENE ZUR BEHANDLUNG EINER ORALEN ERKRANKUNG
PENTACYCLIC TRITERPENES FOR THE TREATMENT OF AN ORAL DISEASE

(30) Priorité: 13.11.2017 FR 1760662
(43) Date de publication de la demande: 23.09.2020
(73) Titulaire: PIERRE FABRE MEDICAMENT, 81500 Lavaur (FR)
(72) Inventeur: MAITRE, Martine, 31170 Tournefeuille (FR); NGUYEN, Thien, 31180 Rouffiac-Tolosan (FR); COUSY, Adrien, 31870 Beaumont Sur Leze (FR); LESTIENNE, Fabrice, 31810 Vernet (FR); STEWARD, Nicolas, 31860 Pins-Justaret (FR)
(74) Mandataire: Regimbeau
(86) Numéro de dépôt international: PCT/EP2018/081076
(87) Numéro de publication internationale: WO 2019/092274

(56) Documents cités:
- KR-A- 20000 060 218
- THANYA K ET AL: "Ethnobotanical approach for oral Lichen Planus - A review", INTERNATIONAL JOURNAL OF DRUG DEVELOPMENT AND RESEARCH OCTOBER-DE CHAUHAN PUBLISHERS IND, vol. 5, no. 4, octobre 2013 (2013-10), pages 54-57, XP002781187, ISSN: 0975-9344
- LIN LI-MEI ET AL: "Comparative observation on the effects of Radix tripterygium hypoglaucum tablet and Tripterygium glycosides tablet in treating erosive oral lichen planus.", CHINESE JOURNAL OF INTEGRATIVE MEDICINE JUN 2005, vol. 11, no. 2, juin 2005 (2005-06), pages 149-150, XP002781188, ISSN: 1672-0415
- WANG YING ET AL: "Celastrol Ameliorates EAE Induction by Suppressing Pathogenic T Cell Responses in the Peripheral and Central Nervous Systems", JOURNAL OF NEUROIMMUNE PHARMACOLOGY, vol. 10, no. 3, septembre 2015 (2015-09), pages 506-516, XP002781189,
- RADHAMANI KANNAIYAN ET AL: "Molecular targets of celastrol derived from Thunder of God Vine: Potential role in the treatment of inflammatory disorders and cancer", CANCER LETTERS, NEW YORK, NY, US, vol. 303, no. 1, 28 octobre 2010 (2010-10-28), pages 9-20, XP028152697, ISSN: 0304-3835, DOI: 10.1016/J.CANLET.2010.10.025 [extrait le 2010-11-01]
- ZHAO LU ET AL: "Effect of non-surgical periodontal therapy on the levels of Th17/Th1/Th2 cytokines and their transcription factors in Chinese chronic periodontitis patients.", JOURNAL OF CLINICAL PERIODONTOLOGY JUN 2011, vol. 38, no. 6, juin 2011 (2011-06), pages 509-516, XP002781190, ISSN: 1600-051X
- WANG LINYUAN ET AL: "Oral Administration of All-Trans Retinoic Acid Suppresses Experimental Periodontitis by Modulating the Th17/Treg Imbalance", JOURNAL OF PERIODONTOLOGY, vol. 85, no. 5, mai 2014 (2014-05), pages 740-750, XP002781191,

## Description

La présente invention est définie dans les revendications jointes ci-après. Elle concerne des compositions comprenant au moins un triterpène pentacyclique pour son utilisation dans le traitement de pathologies bucco-dentaires, notamment des pathologies d'origine infectieuse et/ou inflammatoire de la sphère buccodentaire telles que la gingivite, la parodontite, la péri-mucosite, la péri-implantite ou le lichen plan buccal.

La gingivite est une maladie parodontale causée par la plaque dentaire bactérienne. Lorsqu'elle n'est pas correctement traitée, elle peut évoluer en parodontite entrainant alors des dégâts irréversibles, parmi lesquels la diminution du niveau osseux ou la mobilité des dents.

La péri-mucosite est une inflammation gingivale autour d'un implant, dans le développement de laquelle les bactéries jouent un rôle majeur. En effet, le principal facteur favorisant la mucosité péri-implantaire est la présence de plaque dentaire au niveau de la jonction entre gencive et implant. Lorsqu'elle n'est pas correctement traitée, elle peut évoluer en péri-implantite, caractérisée par une inflammation chronique qui engendre une perte des tissus osseux autour de l'implant, pouvant alors entraîner la perte totale de ce dernier.

Ces pathologies sont distinctes de la gingivite et de la parodontite, qui concernent quant à elles les dents naturelles.

Le lichen plan buccal est une maladie chronique qui peut toucher la peau et la muqueuse orale et qui se traduit par une sensation de brûlure marquée. Les lésions cutanées ont tendance à guérir spontanément. En revanche, les lésions de la cavité bucco-dentaire ont souvent une évolution lente sur plusieurs années.

Les infections microbiennes les plus souvent impliquées dans les pathologies précédemment énumérées sont celles causées par des bactéries Gram-négatif tels que *Porphyromonas gingivalis* et *Fusobacterium nucleatum* ou Gram-positif tel que *Staphylococcus aureus.*

Ces infections induisent dans la cavité buccale des réactions inflammatoires chroniques, une dérégulation du système immunitaire vers une prédominance des lymphocytes T de type TH1/TH17, se traduisant par la synthèse de cytokines pro-inflammatoires telles qu'IL 1, IL 6, IL 17, IL 22, IL 36, IFN-gamma et TNF-alpha, et la synthèse par les fibroblastes gingivaux de métalloprotéases (MMPs). Ces bactéries sont en outre capables de former des biofilms participant ainsi à la formation de la plaque dentaire. L'ensemble de ces facteurs induit une accélération de la dégradation des tissus gingivaux et du déchaussement des dents.

Les traitements actuellement utilisés consistent à traiter l'inflammation par des corticoïdes et à utiliser des antiseptiques (voie buccale) éventuellement en combinaison avec des antibiotiques (voie parentérale) pour limiter la croissance de ces pathogènes. Ces traitements sont lourds, non exempts d'effets secondaires et peuvent en outre générer l'apparition de souches résistantes aux antibiotiques.

Dans la présente invention, les inventeurs ont évalué l'activité bactéricide de certains triterpènes pentacyliques ainsi que d'un extrait de plante de la famille des Celastraceae, le *Tripterygium wilfordii* (TW). Les inventeurs ont pu démontrer que de façon surprenante ces triterpènes pentacyliques et l'extrait testé ont des activités bactéricides fortes et rapides contre S. *aureus* et *F. nucleatum.* En outre, il a pu être démontré que ces triterpènes pentacycliques et cet extrait influaient sur la réponse immunitaire, permettant ainsi de limiter la réaction proinflammatoire. Ils ont, de surcroît, une action directe sur les kératinocytes oraux et la sécrétion des MMPs.

La présente invention concerne donc une composition comprenant au moins un triterpène pentacyclique ou l'un de ses sels pharmaceutiquement acceptables pour son utilisation dans le traitement et/ ou la prévention d'une pathologie bucco-dentaire, dans laquelle le au moins un triterpène pentacyclique est un mélange de Célastrol, de Tingénine A et de Tingénine B.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sel pharmaceutiquement acceptable » d'un composé, un sel qui est pharmaceutiquement acceptable, comme défini ici, et qui possède l'activité pharmacologique souhaitée du composé parent.

Les sels pharmaceutiquement acceptables comprennent notamment :
(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p- toluènesulfonique, l'acide triméthylacétique, l'acide trifluoroacétique et similaires, et
(2) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, l'hydroxyde de sodium et similaires.

Il pourra s'agir d'un sel de sodium lorsque le composé comporte une fonction acide.

### A. Triterpènes pentacycliques et extrait

Par « triterpènes pentacycliques » selon l'invention, on entend les triterpènes pentacycliques naturellement produits par les cellules d'une plante de la famille des Celastraceae et notamment le Célastrol, encore appelé Triptérine (de formule I ci-dessous), la Tingénine A, encore appelée Tingénone ou Mayténine (de formule II ci-dessous), la Tingénine B, encore appelée 22 béta-Hydroxy-tingénone (de formule III ci-dessous), la Pristimérine (de formule IV ci-dessous) et la Tripterygone (de formule V ci-dessous), de préférence le Célastrol, la Tingénine A et la Tingénine B, notamment le Célastrol.

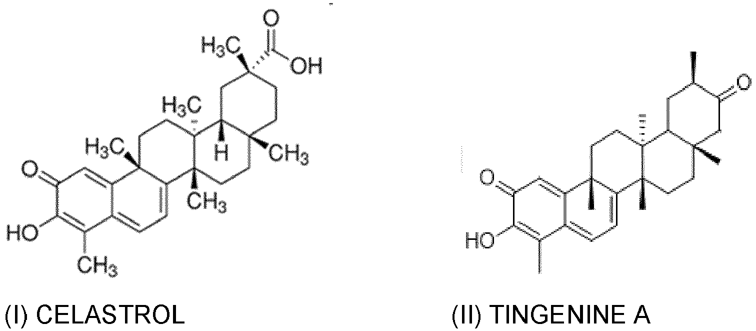

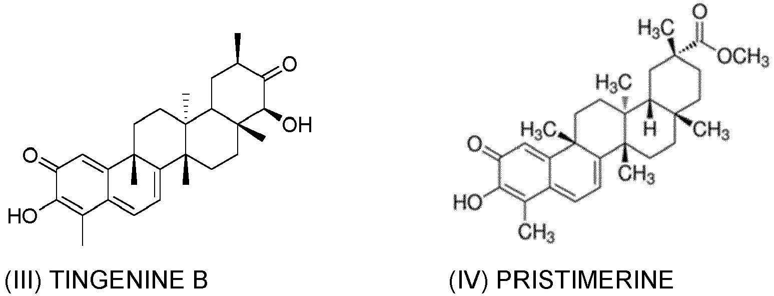

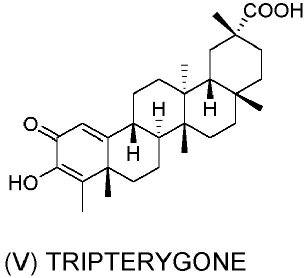

La composition selon l'invention comprend un mélange de Célastrol, de Tingénine A et de Tingénine B ou d'un de leurs sels pharmaceutiquement acceptables.

Dans un mode de réalisation, la composition selon l'invention peut comprendre au moins un triterpène pentacyclique ou l'un de ses sels pharmaceutiquement acceptables sous forme purifiée.

Par « forme purifiée » d'un triterpène pentacyclique ou de l'un de ses sels pharmaceutiquement acceptables, on entend, au sens de la présente invention, un composé sous forme pure c'est-à-dire comprenant au moins 90 % en poids, notamment au moins 95 % en poids, notamment au moins 99 % en poids du composé triterpène pentacyclique, notamment le Célastrol, ou de l'un de ses sels pharmaceutiquement acceptables.

Le Célastrol pourra notamment être obtenu par synthèse chimique notamment telle que décrite dans Camelio et col. (JACS 2015, 137 :11864-867).

Dans un mode de réalisation préféré, le ou les triterpène(s) pentacyclique(s) ou l'un de ses/leurs sels pharmaceutiquement acceptables peut/peuvent être présent(s) dans la composition selon l'invention sous la forme d'un extrait d'une plante de la famille des Celastraceae.

Par « plante de la famille des Celastraceae » selon l'invention, on entend l'ensemble des plantes appartenant à cette famille, et notamment les plantes du genre Tripterygium, Bhesa, Kokkona, Catha, Euonymus, Orthosphenia, Dicarpellum, Celastrus, Maytenus, Peritassa, Siphonodon et Rzedowskia, en particulier Tripterygium, Maytenus et Celastrus. Il s'agit de préférence de plantes telles que *Tripterygium wilfordii, Tripterygium regelii, Tripterygium hypoglaucum, Celastrus orbiculatus* ou *Maytenus ilicifolia,* notamment *Tripterygium wilfordii, Tripterygium hypoglaucum ou Celastrus orbiculatus* notamment *Tripterygium wilfordii.*

Dans un mode de réalisation, cet extrait peut être brut et/ou enrichi en triterpène(s) pentacyclique(s), notamment en Célastrol. L'extrait peut alors comprendre éventuellement des composants (dont des composants actifs) issus de la plante, autres que des triterpènes pentacycliques. Selon l'invention, l'extrait comprend un mélange de Célastrol, de Tingénine A et de Tingénine B, ou d'un de leurs sels pharmaceutiquement acceptables. Selon un mode de réalisation préféré, l'extrait selon l'invention comprend au moins 90%, notamment au moins 95%, en poids, par rapport au poids de l'extrait sec, d'un mélange de Célastrol, de Tingénine A et de Tingénine B, ou d'un de leurs sels pharmaceutiquement acceptables.

De préférence, l'extrait ne contient pas les triterpènes pentacycliques suivants : la Pristimérine, la Tripterygone et leurs sels. De préférence, l'extrait ne contient pas de Triptolide, ni un sel de celui-ci. De manière encore plus préférée, l'extrait ne contient aucun des composants suivants : la Pristimérine, la Tripterygone, le Triptolide et leurs sels.

Le Triptolide répond à la formule ci-dessous :

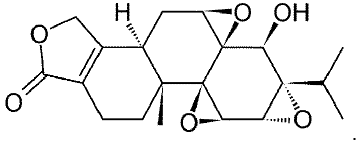

Par « extrait brut », on entend un extrait directement obtenu à partir des plantes.

Par « extrait brut enrichi », on entend un extrait dans lequel la quantité en triterpène(s) pentacyclique(s), notamment en Célastrol, est supérieure à 30 % en poids, notamment supérieur à 50 % en poids par rapport à la quantité en triterpènes pentacycliques, notamment en Célastrol, dans un extrait brut.

Dans un mode de réalisation, l'extrait brut enrichi de la composition pour une utilisation selon l'invention comprend entre 90 % et 100 % de triterpène(s) pentacyclique(s) en poids par rapport au poids total de l'extrait brut enrichi. En particulier, le ou les triterpènes pentacycliques consisteront en un mélange de Célastrol, de Tingénine A et de Tingénine B, ou d'un de leurs sels pharmaceutiquement acceptables. De préférence, l'extrait ne contient aucun des composants suivants : la Pristimérine, la Tripterygone, le Triptolide et leurs sels.

Les extraits purifiés, bruts ou enrichis peuvent être obtenus à partir de n'importe quelle partie des plantes de la famille Celastraceae, notamment les racines, les graines et les parties aériennes.

Alternativement, ils peuvent être obtenus par des cultures de cellules végétales de ces plantes. Dans le cas de cultures de cellules végétales, cet extrait pourra notamment être obtenu à partir du surnageant, de la suspension ou de la biomasse desdites cultures cellulaires (tel que notamment décrit par Coppede et col. (Plant Cell Tiss Organ Cult, 2014 ; 118 :33-43)).

Dans un mode de réalisation, l'extrait brut enrichi est obtenu selon le procédé suivant :
(i) une phase de prolifération de cellules d'une plante de la famille des Celastraceae dans un milieu de prolifération,
(ii) une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) la préparation d'un extrait brut enrichi en triterpènes pentacycliques à partir de la culture cellulaire obtenue à l'étape (ii).

Par « cellules d'une plante de la famille des Celastraceae » selon l'invention, on entend les cellules de n'importe quelle partie de la plante : graines, racines, parties aériennes, et notamment des parties aériennes.

Par « parties aériennes » selon l'invention, on entend les parties de la plante situées au-dessus du sol, par exemple, les feuilles, les tiges, les pétioles et/ou les inflorescences, notamment les feuilles.

Par « phase de prolifération de cellules d'une plante de la famille des Celastraceae », on entend selon l'invention une phase dans laquelle les cellules d'une plante de la famille des Celastraceae sont en suspension dans un milieu de prolifération et dans des conditions adaptées à leur prolifération. Ces cellules pourront notamment être obtenues à partir de cals avant leur mise en suspension. Si nécessaire, les suspensions cellulaires pourront être régulièrement réensemencées afin de les maintenir dans des conditions de prolifération.

Par « cal » selon l'invention, on entend un amas de cellules dédifférenciées, également appelées cellules souches ou cellules méristématiques.

L'induction de cals pourra être obtenue par toute méthode connue de l'homme du métier. Les cals selon l'invention pourront notamment être obtenues de la manière décrite ci-après.

L'induction de cals à partir d'un explant de tissu de partie de plante, notamment une partie aérienne, de la famille des Celastraceae, par exemple de *Tripterygium wilfordii,* est bien connue de l'homme du métier.

L'induction de cals pourra notamment être réalisée par :
- obtention d'un explant de tissu de la plante, par exemple un morceau de feuille de taille de 1 cm² environ,
- mise en culture de l'explant sur un milieu de prolifération selon l'invention gélosé (par exemple par ajout de 4 à 12 g/L d'agar, par exemple environ 8 g/L d'agar, au milieu de prolifération selon l'invention),
- incubation, notamment à l'obscurité, à une température d'environ 25-30 °C, par exemple à environ 27 °C à 28 °C.

### Etape (i) : phase de prolifération de cellules

L'homme du métier connaissant les cultures de cellules d'une plante de la famille des Celastraceae pourra facilement déterminer la composition du milieu de prolifération nécessaire à leur prolifération. Préférentiellement, ce milieu de prolifération permettra la prolifération des cellules sous formes dédifférenciées, c'est-à-dire sous formes totipotentes. Le maintien sous forme dédifférenciée pourra notamment être obtenu par l'utilisation de ratios particuliers cytokinines/auxines dans le milieu de prolifération.

Le « milieu de prolifération » selon l'invention pourra notamment comprendre :
- au moins un macroélément, notamment choisi parmi NH₄NO₃, KNOs, CaCl₂.2H₂O, MgSO₄.7H₂O, KH₂PO₄ et un mélange de ceux-ci, par exemple à une concentration en macroéléments totale comprise entre 1000 et 9000 mg/L, par exemple entre 3000 et 8000 mg/L de milieu de prolifération : lors de la phase de prolifération, la concentration en macroéléments totale du milieu de prolifération sera notamment comprise entre 3000 et 5500 mg/L de milieu prolifération ;
- au moins un microélément, notamment choisi parmi KI, H₃BO₃, MnSO₄.4H₂O, ZnSO₄.H₂O, Na₂MoO₄.2H₂O, CuSO₄.5H₂O, CoCl₂.6H₂O, FeSO₄.7H₂O, Na₂EDTA.2H₂O et un mélange de ceux-ci, par exemple à une concentration en microéléments totale comprise entre 10 et 200 mg/L, notamment entre 50 et 150 mg/L de milieu prolifération ;
- au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3 g/L, notamment de 0,05 à 1 g/L de milieu de prolifération ;
- au moins un acide aminé, notamment la glycine, par exemple à une concentration en acide aminés totale pouvant aller de 0,15 à 5 mg/L, notamment entre 1 et 4 mg/L de milieu de prolifération : lors de la phase de prolifération, la concentration en acide aminés du milieu de prolifération sera notamment comprise entre 1 et 2,5 mg/L de milieu prolifération ;
- au moins une source de carbone, notamment du saccharose, par exemple à une concentration totale en source de carbone de 10 à 70 g/L de milieu de prolifération, par exemple à environ 30 g/L ;
- au moins une hormone végétale (encore appelée hormone de croissance végétale ou facteur de croissance végétale ou régulateur de croissance végétale) notamment choisie parmi une ou plusieurs cytokinines, notamment la kinétine et/ou la 6-furfurylaminopurine, une ou plusieurs auxines, notamment l'acide 2,4-dichlorophénoxyacétique (2,4D) et/ou l'acide naphthalène acétique (NAA), et un mélange de celles-ci. Lors de la phase de prolifération le milieu de prolifération comprendra notamment au moins une cytokinine et au moins une auxine.

Les hormones de croissance végétale seront notamment ajoutées au milieu de prolifération à une concentration et à un ratio permettant la prolifération des cellules sous formes dédifférenciées. Elles seront notamment choisies parmi la kinétine, la 6-furfurylaminopurine, l'acide 2,4-dichlorophénoxyacétique (2,4D), l'acide naphthalène acétique (NAA) et un mélange de ceux-ci ; notamment choisies parmi la kinétine, l'acide 2,4-dichlorophénoxyacétique (2,4D), l'acide naphthalène acétique (NAA) et un mélange de ceux-ci. Il pourra s'agir en particulier d'un mélange de kinétine, acide 2,4-dichlorophénoxyacétique (2,4D) et acide naphthalène acétique.

La concentration en auxines selon l'invention sera notamment comprise entre 0,001 et 10 mg/L de milieu de prolifération, par exemple entre 0,1 et 3 mg/L de milieu de prolifération.

La concentration en cytokinines selon l'invention sera notamment comprise entre 0,01 et 0,5 mg/L de milieu de prolifération, notamment entre 0,05 et 0,15 mg/L.

Dans un mode de réalisation, le ratio hormonal auxines / cytokinines sera compris entre 0,2 à 2,5/0,01 à 0,5, notamment entre 1 à 2/ 0,05 à 0,2 notamment sera d'environ 1,5/0,1.

Notamment le milieu de de prolifération selon l'invention pourra comprendre 1,5 mg/L d'auxine, notamment d'acide 2,4-dichlorophénoxyacétique (2,4D) et d'acide naphthalène acétique (NAA), et 0,1 mg/L de cytokinine, notamment de kinétine.

Le milieu de prolifération sera stérile et préférentiellement à un pH proche de la neutralité.

Un exemple de milieu de prolifération adapté à la prolifération des cellules d'une plante de la famille des Celastraceae selon l'invention est notamment décrit par Murashige & Skoog (1962) ou dans les exemples de la présente demande.

Ce milieu de prolifération peut par exemple avoir la composition suivante (les concentrations sont exprimées par rapport au volume de milieu de prolifération sans cellule) : Macroéléments : NH₄NO₃ à 1 650 mg/L, KNOs à 1 900 mg/L, CaCl₂.2H₂O à 440 mg/L, MgSO₄.7H₂O à 370 mg/L, KH₂PO₄ à 170 mg/L ; Microéléments : KI à 0,83 mg/L, H₃BO₃ à 6,2 mg/L, MnSO₄.4H₂O à 22,3 mg/L, ZnSO₄.H₂O à 6,6 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 27,8 mg/L, Na₂EDTA.2H₂O à 37,3 mg/L ; Vitamines: myo-inositol à 100 mg/L, acide nicotinique à 0,5 mg/L, pyridoxine-HCl à 0,5 mg/L, thiamine-HCl à 0,5 mg/L ; Acides aminés : glycine à 2 mg/L ; Source de carbone : saccharose à 30 g/L ; et Hormones végétales : acide naphtalène acétique à 1 mg/L, acide 2,4-dichlorophénoxyacétique à 0,5 mg/L, kinétine à 0,1 mg/L, le tout ajusté à pH 6 avant stérilisation (par exemple par autoclavage de 20 min à 121 °C ou par filtration sur filtre de 0,2 µm).

Alternativement, le milieu de prolifération peut avoir la composition suivante : (les concentrations sont exprimées par rapport au volume de milieu de prolifération sans cellule): Macroéléments: NH₄NO₃ à 1650 mg/L, KNOs à 2500 mg/L, CaCl₂.2H₂O à 440 mg/L, MgSO₄.7H₂O à 370 mg/L, KH₂PO₄ à 130 mg/L ; Microéléments : KI à 0,41 mg/L, H₃BO₃ à 6,2 mg/L, MnSO₄.4H₂O à 22,3 mg/L, ZnSO₄.H₂O à 7,5 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 19,85 mg/L, Na₂EDTA.2H₂O à 26,64 mg/L ; Vitamines : myo-inositol à 50 mg/L, acide nicotinique à 0,25 mg/L, pyridoxine-HCl à 0,25 mg/L, thiamine-HCl à 0,25 mg/L ; Source de carbone : saccharose à 30 g/L ; et Hormones végétales : kinétine à 0,083 mg/L, acide 2,4-dichlorophénoxyacétique (2,4D) à 0,575 mg/L, acide naphthalène acétique (NAA) à 0,350 mg/L.

Alternativement, le milieu de prolifération selon l'invention peut avoir la composition suivante (les concentrations sont exprimées par rapport au volume de milieu de prolifération sans cellule): NH₄NO₃ à 20 mM, KNOs à 19 mM, CaCl₂.2H₂O à 3 mM, MgSO₄.7H₂O à 1,50 mM, KH₂PO₄ à 1,2 mM, KI à 0,005 mM, H₃BO₃ à 0,1 mM, MnSO₄.4H₂O à 0,1 mM, ZnSO₄.H₂O à 0,04 mM, Na₂MoO₄.2H₂O à 0,001 mM CuSO₄.5H₂O à 0,0001 mM, CoCl₂.6H₂O à 0,0001 mM, FeSO₄.7H₂O à 0,1 mM, Na₂EDTA.2H₂O à 0,1 mM, myo-inositol 0,5 mM, acide nicotinique à 0,004 mM, pyridoxine-HCl à 0,002 mM, thiamine-HCl à 0,0015 mM, glycine à 0,03 mM, saccharose à 87,6 mM, acide naphtalène acétique à 0,005 mM, acide 2,4-dichlorophénoxyacétique à 0,002 mM, et kinétine à 0,0005 mM.

L'ensemencement du milieu de prolifération pourra être réalisé à partir de la mise en suspension de cellules de cals à une concentration comprise entre 20 et 300 g dans 1L de milieu de prolifération et préférentiellement entre 100 et 200 g dans 1L de milieu de prolifération, par exemple environ 150 g dans 1L de milieu de prolifération.

La phase de prolifération aura lieu dans des conditions de multiplication de la biomasse. Par « conditions de multiplication de la biomasse » selon l'invention, on entend notamment les conditions de température, de durée, d'agitation et de luminosité nécessaires à la prolifération des cellules en suspension. L'homme du métier connaissant les cultures de cellules d'une plante de la famille de Celastraceae pourra facilement déterminer les conditions de multiplication de la biomasse. Dans un mode de réalisation selon l'invention, l'étape de prolifération de la biomasse se fera à l'obscurité, à une température comprise entre 20 et 35 °C, notamment entre 27 et 28 °C, notamment à environ 27 ou 28 °C, en particulier sous une agitation comprise entre 100 et 200 rpm, notamment à environ 125 rpm (orbitale de 22,5 mm) et pendant une durée comprise entre 10 et 30 jours, notamment 15 jours de culture.

Au cours de cette étape, les cellules peuvent être « repiquées » ou propagées, par exemple tous les 7 à 15 jours. Le repiquage des cellules est bien connu par l'homme du métier, il pourra notamment consister à diluer une partie de la culture cellulaire dans du milieu neuf concentré. Par exemple, 1/5^{ème} de la culture est remise en suspension dans un volume de milieu neuf correspondant au volume de la culture initiale. Il permet l'entretien de la lignée cellulaire en milieu liquide dans un état de prolifération.

### Etape (ii) - phase d'élicitation

Après la phase de prolifération, les cellules obtenues dans la phase (i) sont élicitées par ajout d'un cocktail d'élicitation. Le milieu de prolifération auquel a été ajouté le cocktail d'élicitation sera nommé « milieu d'élicitation » selon la présente invention.

La phase d'élicitation est la phase dans laquelle les cellules sont maintenues dans un état physiologique favorisant la biosynthèse de métabolites secondaires tels que les triterpènes pentacycliques.

La production de triterpènes pentacycliques, notamment de Célastrol, a lieu, au cours de la phase d'élicitation, dans le cytosol de la cellule et peut diffuser en partie dans le milieu d'élicitation. La phase d'élicitation au sens de la présente invention correspond donc à la phase de production (biosynthèse) du Célastrol et de ses dérivés (triterpènes pentacycliques).

L'élicitation se fera préférentiellement après une phase de prolifération de 7 à 21 jours, notamment 12 à 20 jours, notamment 15, 16 ou 17 jours (notamment sans repiquage). Alternativement l'ajout du cocktail d'élicitation pourra se faire lorsque la concentration en cellules obtenue lors de la phase de prolifération est double, notamment supérieure au double, par rapport à la concentration initiale en cellules dans le milieu de prolifération. L'ajout du cocktail d'élicitation pourra notamment se faire lorsque la concentration en cellule est supérieure à 200 g/L, par exemple entre 200 et 400 g/L, notamment environ 300 g/L (en quantité de cellules par litre de milieu de prolifération). Dans un mode de réalisation, l'ajout du cocktail d'élicitation se fera dans une culture dont la concentration en cellules est passée de 150 à 200 g/L lors du début de la phase de prolifération à une concentration comprise entre 300 et 400 g/L lors de la fin de la phase de prolifération.

A la suite de la phase de prolifération, les cellules végétales ont consommé la plupart des, voire tous les éléments contenus dans le milieu de prolifération, et en particulier les sources de carbone telles que le saccharose. Il peut donc être nécessaire de rétablir la composition du milieu avant ou en même temps que l'ajout du cocktail d'élicitation.

Pour rétablir la composition du milieu, on peut notamment concentrer la culture cellulaire obtenue après l'étape de prolifération, par exemple par décantation ou filtration puis ajouter du milieu de prolifération neuf aux cellules ainsi obtenues. Dans ce cas, les cellules seront re-suspendues dans le milieu de prolifération neuf de manière à obtenir une concentration comprise entre 200 g/L et 400 g/L, par exemple entre 250 et 350 g/L, notamment d'environ 300 g/L (en quantité de cellules par litre de milieu de prolifération).

Alternativement, on peut ajouter dans la culture cellulaire un mélange concentré permettant de rétablir les concentrations des éléments du milieu de prolifération. Le mélange concentré sera notamment ajouté juste avant, juste après ou en même temps que le cocktail d'élicitation. Par exemple on peut remplacer 1/5^{ème} de la culture cellulaire par un volume équivalent du milieu de prolifération concentré 5 fois.

On considère que la concentration en éléments du milieu de prolifération est proche ou équivalente à zéro après 14, 15 ou 16 jours de phase de prolifération. En particulier on considère que la concentration en éléments minéraux (plus particulièrement les macroéléments et les microéléments) et carbonés (plus particulièrement les sources de carbone) est proche de ou équivalente à zéro après 14, 15 ou 16 jours de phase de prolifération.

Dans un mode de réalisation, le milieu de prolifération selon l'invention au début de la phase d'élicitation comprendra entre autres :
- au moins un macroélément, notamment choisi parmi NH₄NO₃, KNOs, CaCl₂.2H₂O, MgSO₄.7H₂O, KH₂PO₄ et un mélange de ceux-ci, par exemple à une concentration de macroéléments totale comprise entre 5 000 et 8 000 mg/L, préférentiellement supérieure à 6 000 mg/L de milieu de prolifération, avantageusement entre 6 000 et 8 000 mg/L ;
- au moins un microélément, notamment choisi parmi KI, H₃BO₃, MnSO₄.4H₂O, ZnSO₄.H₂O, Na₂MoO₄.2H₂O, CuSO₄.5H₂O, CoCl₂.6H₂O, FeSO₄.7H₂O, Na₂EDTA.2H₂O et un mélange de ceux-ci, par exemple à une concentration en microéléments totale comprise entre 10 et 200 mg/L, notamment entre 50 et 150 mg/L de milieu prolifération ;
- au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3 g/L, notamment de 0,05 à 1 g/L de milieu de prolifération ;
- au moins un acide aminé, notamment la glycine, par exemple à une concentration dans le milieu de prolifération comprise entre 3 et 4 mg/L de milieu de prolifération ;
- au moins une source de carbone, notamment du saccharose, par exemple à une concentration totale en source de carbone de 10 à 70 g/L de milieu de prolifération, par exemple à environ 30 g/L.

Dans un mode de réalisation le milieu de prolifération au début de la phase d'élicitation ne comprendra pas, où comprendra une quantité négligeable, notamment moins de 0,001 g/ml, de cytokinine et d'auxine.

Le milieu de prolifération lors de la phase d'élicitation sera avantageusement stérile et préférentiellement à un pH proche de la neutralité.

Le milieu de prolifération lors de la phase d'élicitation pourra notamment avoir la composition suivante : Macroéléments : NH₄NO₃ à 2,8 g/L, KNOs à 3 g/L, CaCl₂.2H₂O à 0,45 g/L, MgSO₄.7H₂O à 74 mg/L, KH₂PO₄ à 34 mg/L ; Microéléments : KI à 0,16 mg/L, H₃BO₃ à 6.2 mg/L, MnSO₄.4H₂O à 18,5 mg/L, ZnSO₄.H₂O à 6,6 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 28 mg/L, Na₂EDTA.2H₂O à 37 mg/L ; Vitamines : myo-inositol à 250 mg/L, acide nicotinique à 1,7 mg/L, pyridoxine-HCl à 1 mg/L, thiamine-HCl à 1 mg/L ; Acide aminé : glycine à 4 mg/L ; Source de carbone : saccharose à 30 g/L.

Par « cocktail d'élicitation » selon l'invention, on entend un cocktail permettant de stopper la division cellulaire. Ce cocktail d'élicitation comprend au moins un éliciteur de type composé monocarboxylique et au moins un éliciteur biotique. Le cocktail d'élicitation est introduit, par exemple, à l'aide de solutions mères concentrées dans le milieu de culture.

Par « éliciteur de type composé monocarboxylique » selon l'invention, on entend plus particulièrement un éliciteur choisi dans le groupe comprenant, de préférence constitué par, l'acide 5-chloro salicylique (5-Chloro SA), l'acide salicylique (SA), l'acide acétyl-salicylique (ASA), un ester de méthyle, notamment le jasmonate de méthyle (MeJa), et un mélange de ceux-ci. Dans un mode de réalisation selon l'invention, l'éliciteur de type composé monocarboxylique est le jasmonate de méthyle, l'acide salicylique et/ou l'acide 5-chloro salicylique, notamment le jasmonate de méthyle. L'éliciteur de type composé monocarboxylique sera notamment ajouté de façon à obtenir une concentration finale comprise entre 0,005 et 0,1 g/L, notamment 0,01 et 0,05 g/L de milieu d'élicitation, notamment de 0,002 et 0,004 g/L de milieu d'élicitation.

Par « éliciteur biotique » selon l'invention, on entend plus particulièrement un éliciteur biotique choisi dans le groupe comprenant, de préférence constitué par, une N-acétylaminoglucosamine, notamment la chitine, le chitosan, les extraits de microorganismes ou de champignons, les oligosaccharides (polysaccharides, pectines, cellulose). Dans un mode de réalisation, l'éliciteur biotique est la chitine. La chitine est un polymère linéaire de motif répétitif : béta-1,4 N-acétyl D-glucosamine. L'éliciteur biotique sera notamment ajouté de façon à obtenir une concentration finale comprise entre 0,05 et 50 g/L de milieu d'élicitation, par exemple de 0,1 à 10 g/L, par exemple de 0,5 à 7 g/L, notamment de 1 à 5 g/L de milieu de d'élicitation.

Dans un mode de réalisation, le cocktail d'élicitation comprend du jasmonate de méthyle, notamment à une concentration finale dans le milieu d'élicitation entre 0,002 et 0,005 g/L, et de la chitine, notamment à une concentration finale entre 1 et 4 g/L de milieu d'élicitation.

Dans un mode de réalisation, le cocktail d'élicitation selon l'invention comprendra en outre, au moins un facteur de différenciation cellulaire des cellules végétales et/ou au moins un précurseur de la voie de synthèse des terpènes.

Le « facteur de différenciation cellulaire des cellules végétales » selon l'invention pourra notamment être choisi dans le groupe comprenant, de préférence constitué par, une cytokinine, notamment la benzyl-aminopurine (BAP), l'acide abscissique, la kinétine, le thidiazuron, la 6-γ,γ-diméthylallylaminopurine (2iP ou isopentényladénine) ou la zéatine, une gibbérelline et un mélange de celles-ci, notamment la BAP et/ou la 2iP, en particulier la 2iP.

Le « précurseur de synthèse des terpènes » selon l'invention pourra notamment être choisi dans le groupe comprenant, de préférence constitué par, le pyruvate de sodium ; le pyrophosphate de potassium ; l'acide mévalonique ; le géraniol ; le farnésol ; l'isopentényle, le diméthylallyle, y compris leurs formes pyrophosphatées ; l'acétate de sodium ; l'acide pyruvique et leurs mélanges, notamment le géraniol, le farnésol, le pyruvate de sodium, le pyrophosphate de potassium et leurs mélanges, tel que le pyruvate de sodium et/ou le pyrophosphate de potassium.

La BAP pourra notamment être utilisée à une concentration finale dans le milieu d'élicitation comprise entre 0,01 et 5 mg/L, par exemple entre 0,5 et 5 mg/L de milieu d'élicitation.

L'acide 5-chloro salicylique (5-Chloro SA) pourra notamment être utilisé à une concentration finale dans le milieu d'élicitation comprise entre 0,1 et 15 mg/L.

L'acide salicylique pourra notamment être utilisé à une concentration finale dans le milieu d'élicitation comprise entre 0,1 et 100 mg/L, par exemple entre 20 et 60 mg/L, par exemple environ 45 mg/L.

Le farnésol pourra notamment être utilisé à une concentration finale dans le milieu d'élicitation de 1 à 100 mg/L, par exemple de 15 à 30 mg/L, par exemple à environ 30 mg/L.

Le géraniol pourra notamment être utilisé à une concentration finale dans le milieu d'élicitation de 1 à 100 mg/L, par exemple de 20 à 30 mg/L.

Le pyruvate de sodium pourra notamment être utilisé à une concentration finale dans le milieu d'élicitation de 100 à 5 000 mg/L, par exemple de 500 à 2 000 mg/L.

Le pyrophosphate de potassium pourra notamment être utilisé à une concentration finale dans le milieu d'élicitation de 1 à 2 000 mg/L, par exemple de 100 à 1 000 mg/L de milieu d'élicitation.

La 2iP pourra notamment être utilisée à une concentration finale dans le milieu d'élicitation de 0,005 à 10mg/L, par exemple de 0,01 mg/L à 3 mg/L, par exemple de 0,1 à 2 mg/L.

Dans un mode de réalisation de l'invention, le cocktail d'élicitation comprend du jasmonate de méthyle, de la chitine, du pyruvate de sodium, du pyrophosphate de potassium ou un mélange de ceux-ci.

Dans un mode de réalisation de l'invention, le cocktail d'élicitation comprend du jasmonate de méthyle, de la chitine, du pyruvate de sodium, du pyrophosphate de potassium, et éventuellement de la BAP et/ou de la 2iP.

Dans un mode de réalisation de l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2 000 mg/L), du pyrophosphate de potassium (de 100 à 1 000 mg/L), de la 2iP (de 0,1 à 2 mg/L), du jasmonate de méthyle (de 0,002 à 0,005 g/L) et de la chitine (de 1 à 4 g/L).

Dans un autre mode de réalisation, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) de la benzyl-aminopurine (BAP) (de 0,5 à 5 mg/L, notamment de 0,5 à 3 mg/L) ; de l'acide 5-chloro salicylique (5-Chloro SA) (de 2 à 6 mg/L, notamment de 3 à 5 mg/L, par exemple à environ 3 ou à environ 5 mg/L), de l'acide acétyl-salicylique (ASA) et/ou de l'acide salicylique (SA) (de 20 à 60 mg/L, notamment de 30 à 50 mg/L, notamment de 33 à 45 mg/L) ; du jasmonate de méthyle (MeJA) (de 0,002 à 0,005 g/L, notamment de 10 à 40 mg/L) ; de la chitine (de 1 à 4 g/L) ; et du farnésol (F-OH) (de 19 à 40 mg/L) et/ou du géraniol (de 20 à 30 mg/L).

Dans un autre mode de réalisation le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2 000 mg/L), du pyrophosphate de potassium (de 100 à 1 000 mg/L), de la 2iP (de 0,1 à 1 mg/L), du jasmonate de méthyle (MeJA) (de 0,002 à 0,005 g/L, notamment de 10 à 40 mg/L) et de la chitine (de 1 à 4 g/L).

Dans un autre mode de réalisation le cocktail d'élicitation comprend ou consiste en (les concentrations données entre parenthèses correspondent à la concentration dans le milieu d'élicitation, le cocktail pouvant être plus ou moins concentré en fonction de la dilution envisagée) du pyruvate de sodium (environ 1,5 g/L), du pyrophosphate de potassium (environ 0,4 g/L), du 2iP (environ 0,4 mg/L), du jasmonate de méthyle (MeJA) (environ 0,03 mg/L), de la chitine (environ 2 g/L).

Pendant la phase d'élicitation, après ajout du cocktail d'élicitation, la culture est maintenue sous agitation, notamment entre 50 et 200 rpm, notamment à environ 125 rpm, pendant une période comprise entre 3 et 30 jours, notamment entre 10 et 25 jours, notamment de 12 à 15 jours, en particulier à une température comprise entre 20 et 35 °C, notamment à environ 27 °C et avantageusement avec un taux d'oxygène dissout dans le milieu de culture de 2 à 40 %, préférentiellement à environ 16 %. Un apport en air stérile suffisamment enrichi en oxygène pourra être fourni si nécessaire notamment dans le volume mort du bioréacteur ou par diffusion dans le milieu. De préférence, la phase d'élicitation (iii) est menée à l'obscurité. Lors de la phase d'élicitation, la culture cellulaire n'est préférentiellement pas repiquée.

### Etape (iii) - phase de préparation de l'extrait brut enrichi en triterpènes pentacycliques

Après la phase d'élicitation, le procédé comprend une étape de préparation d'un extrait brut enrichi en triterpènes pentacycliques, notamment le Célastrol.

L'extrait brut enrichi pourra notamment être obtenu par séparation de la biomasse et du surnageant de culture. La séparation pourra notamment être faite par filtration directe (0-50 µm), par centrifugation ou par décantation des cellules.

Dans un mode de réalisation, l'extrait brut enrichi compris dans la composition pour une utilisation selon l'invention pourra consister en le surnageant de culture ainsi récupérée.

L'extrait brut enrichi pourra également être obtenu après la lyse de la biomasse. Par exemple, les cellules contenues dans la biomasse récupérée pourront être lysées par une méthode physique (sonication ou broyage) ou chimique (lyse acide) puis ce lysat sera soumis à une extraction par solvant. La phase organique, comprenant notamment les triterpènes issus du cytosol, est ensuite récupérée, notamment par décantation ou centrifugation. Le solvant sera notamment un solvant de type ester, et plus particulière un acétate d'alkyle, l'alkyle étant plus particulièrement linéaire ou ramifié à 1 à 6 atomes de carbone, notamment l'acétate d'éthyle ou l'acétate d'isopropyle. Le volume de solvant utilisé sera notamment de 2 volumes par poids de biomasse.

Préférentiellement, l'extrait brut enrichi de la composition pour une utilisation selon l'invention correspondra à la phase organique ainsi récupérée.

Alternativement, l'extrait brut enrichi pourra être obtenu après évaporation du solvant et sa substitution notamment par un support adapté au domaine d'utilisation de l'extrait (cosmétique, pharmaceutique) et notamment les huiles végétales ou des solvants tel que notamment le Pentylène Glycol (ou Pentiol), ou du Myritol 3180.

L'extrait brut enrichi ainsi obtenu comprendra avantageusement un ou plusieurs triterpènes pentacycliques choisis parmi le Célastrol, la Tingénine A, la Tingénine B, et leurs sels pharmaceutiquement acceptables, de préférence consistera en un mélange de Célastrol, de Tingénine A et de Tingénine B, ou d'un de leurs sels pharmaceutiquement acceptables.

De préférence, cet extrait brut enrichi ne contient pas les triterpènes pentacycliques suivants : la Pristimérine, la Tripterygone et leurs sels. De préférence, cet extrait brut enrichi ne contient pas de Triptolide, ni un sel de celui-ci. De manière encore plus préférée, cet extrait brut enrichi ne contient aucun des composants suivants : la Pristimérine, la Tripterygone, le Triptolide et leurs sels.

L'extrait brut enrichi n'est pas purifié. L'extrait enrichi brut pourra éventuellement être purifié en une étape ultérieure.

Lorsque l'extrait brut enrichi est purifié, pour donner un extrait purifié, le procédé selon l'invention comprend en outre une étape (iv) d'obtention d'un extrait purifié d'un ou plusieurs triterpènes pentacycliques à partir de l'extrait enrichi brut obtenu à l'étape (iii).

L'extrait purifié selon l'invention comprendra alors plus de 90 % ou 90%, notamment plus de 95 % ou 95%, notamment plus de 98 % ou 98% en poids d'un ou plusieurs triterpènes pentacycliques selon l'invention par rapport au poids total de l'extrait, plus particulièrement de l'extrait sec. En particulier, le ou les triterpènes pentacycliques consisteront en un mélange de Célastrol, de Tingénine A et de Tingénine B, ou d'un de leurs sels pharmaceutiquement acceptables. De préférence, l'extrait ne contient aucun des composants suivants : la Pristimérine, la Tripterygone, le Triptolide et leurs sels.

Dans un mode de réalisation, l'extrait purifié selon l'invention comprend plus de 60 % ou 60%, notamment plus de 80% ou 80%, notamment plus de 90% ou 90% notamment plus de 95 % ou 95%, notamment plus de 98 % ou 98%, notamment 100 % de Célastrol en poids par rapport au poids total de l'extrait.

Dans un mode de réalisation, l'extrait purifié selon l'invention comprend entre 1 et 20%, notamment entre 5 et 15 %, notamment entre 8 et 12 % de Tingénine A en poids par rapport au poids total de l'extrait.

Dans un mode de réalisation, l'extrait purifié selon l'invention comprend entre 1 et 20 %, notamment entre 5 et 15 %, notamment entre 8 et 12 % de Tingénine B en poids par rapport au poids total de l'extrait.

La purification de l'extrait enrichi selon l'invention pourra notamment être réalisée par une phase de séparation du ou des triterpène(s) pentacyclique(s), notamment le Célastrol, la Tingénine A et/ou la Tingénine B, notamment par fractionnement HPLC (chromatographie liquide à haute performance), au cours duquel les pics à 426 nm comprenant le Célastrol, la Tingénine A et la Tingénine B sortent respectivement à 19,75 min, 18,03 min et 16,21 min. La purification du Célastrol pourra notamment être effectuée comme décrit dans les exemples de la présente demande.

L'extrait selon l'invention aura ainsi avantageusement une grande pureté. L'extrait sec pourra être constitué essentiellement de Célastrol, de Tingénine A et de Tingénine B. Ainsi, il sera dépourvu de tout composé toxique pour une utilisation thérapeutique.

### B. Composition

Dans un mode de réalisation, la composition pour une utilisation selon l'invention comprend du Célastrol entre 0,002 et 10 % en poids par rapport au poids de la composition totale, notamment entre 0,0005 et 5 %, notamment entre 0,5 et 1 %.

Dans un mode de réalisation, la composition pour une utilisation selon l'invention comprend de la Tingénine A entre 0,002 et 10 % en poids par rapport au poids de la composition totale, notamment entre 0,0005 et 5 %, notamment entre 0,5 et 1 %.

Dans un mode de réalisation, la composition pour une utilisation selon l'invention comprend la Tingénine B entre 0,002 et 10 % en poids par rapport au poids de la composition totale, notamment entre 0,0005 et 5 %, notamment entre 0,5 et 1 %.

Dans un mode de réalisation, la composition pour une utilisation selon l'invention comprend entre 0,002 et 10 % en poids de triterpène(s) pentacyclique(s) par rapport au poids de la composition totale, notamment entre 0,0005 et 5 %, notamment entre 0,5 et 1 %, le ou les triterpène(s) pentacyclique(s).

Dans un mode de réalisation, la composition pour une utilisation selon l'invention comprend entre 0,002 et 10 % d'un extrait brut ou brut enrichi en Célastrol en poids par rapport au poids de la composition totale, notamment entre 0,0005 et 5 %, notamment entre 0,5 et 1 %.

Dans un mode de réalisation, la composition pour une utilisation selon l'invention comprend entre 0,5 % et 2 % de Célastrol, entre 0,01 % et 1 % de Tingénine A et/ou entre 0,01 et 1 % de Tingénine B.

Dans un mode de réalisation, la composition pour une utilisation selon l'invention comprend entre 0,002 et 10 % de pentylène glycol en poids par rapport au poids de la composition totale, notamment entre 0,0005 et 5 %, notamment entre 0,5 et 1 %.

La composition pour son utilisation selon l'invention pourra en outre comprendre un ou plusieurs excipients pharmaceutiquement acceptables.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

La composition pour une utilisation selon la présente invention peut notamment se présenter sous la forme d'un bain de bouche, d'un gel ou d'une pâte dentifrice, d'un gel gingival, d'une gomme à mâcher, d'une pastille à sucer, d'un fil dentaire, d'une capsule extrudée, de brins pour brosses à dents, d'un spray, d'un pansement adhésif, d'une lingette imprégnée, d'une pâte adhésive pour dentiers et prothèses ou d'une pâte prophylactique de polissage. Dans un mode de réalisation, la composition pour une utilisation selon l'invention est sous forme d'une pâte dentifrice ou d'un bain de bouche.

Dans un mode de réalisation, la composition pour une utilisation selon l'invention est sous forme de dentifrice (autrement appelle pâte dentifrice). Elle peut alors comprendre tout excipient pharmaceutiquement acceptable bien connu de l'homme du métier comme apte à entrer dans ce type de composition.

La composition sous forme de dentifrice pourra notamment comprendre : un agent de type moussant tel qu'un sulfate, en particulier le laurylsulfate de sodium, des enzymes, des vitamines, des minéraux tel le calcium, des arômes, des agents polissants tels que des silices, du bicarbonate de sodium ou des phosphates de calcium, des conservateurs, des colorants, des agents épaississants.

La composition notamment sous forme de dentifrice ou de bain de bouche pourra en outre comprendre d'autres principes actifs comme des agents antibactérien, tels que le triclosan ou la chlorexidine ou agents anti-biofilm tel qu'un extrait de canneberge (ou Vaccinium macrocarpon).

Dans un mode de réalisation, la composition pour une utilisation selon l'invention ne comprend pas d'autre principe actif que ceux contenus dans l'extrait de *Tripterygium wilfordii,* notamment que le Célastrol, la Tingénine A et/ou la Tingénine B.

### C. Utilisation

Par « pathologie bucco-dentaire » selon l'invention on entend notamment toute pathologie liée à un déséquilibre du microbiote buccal, et notamment toutes pathologies induites ou entretenues par la présence de bactéries pathogènes, notamment de *S*. *aureus* et/ou de *F. nucleatum.* Les pathologies bucco-dentaires selon l'invention sont aussi des pathologies médiées par les cytokines et les chémiokines de type Th17.

Parmi ces pathologies on pourra notamment citer la parodontite, la gingivite, la péri-mucosite, la péri-implantite et le lichen plan buccal (ou oral).

Par « traitement » selon l'invention on entend l'inhibition de l'évolution, plus particulièrement la régression, préférentiellement la disparition de la pathologie bucco-dentaire.

Par « prévention » selon l'invention, on entend empêcher ou retarder l'apparition de la pathologie bucco-dentaire selon l'invention.

La composition pour une utilisation selon l'invention présentera une activité bactéricide et ou bactériostatique notamment sur S. *aureus* et F. *nucleatum.* Cette activité pourra être mesurée notamment par calcul de la CMI (Concentration Minimale Inhibitrice) et de la CMB (Concentration Minimale Bactéricide) par toutes les méthodes bien connues de l'homme du métier et notamment tel que décrit dans les exemples de la présente demande. Par activité bactéricide ou bactériostatique sur S. *aureus* et *F. nucleatum* on entend notamment au sens de la présente invention que la CMI et/ou la CMB de la composition selon l'invention est inférieure de 2 à 100 fois par rapport à la CMI et à la CMB d'une composition témoin ne comprenant pas de triterpène pentacyclique.

Dans un mode de réalisation, cette activité bactéricide sera obtenue en moins de 60 minutes, notamment moins de 15 minutes, notamment moins de 5 minutes, notamment moins de 3 minutes. La cinétique d'activité de la composition pourra notamment être mesurée comme décrit dans les exemples. Ces temps particulièrement courts permettent d'envisager une utilisation en soin quotidien bucco-dentaire.

### FIGURES :

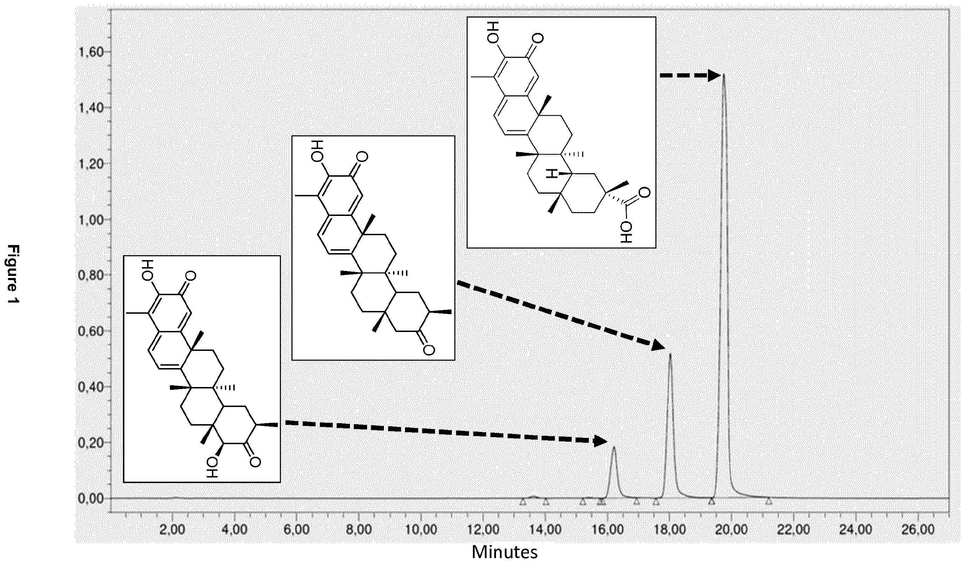

**Figure 1** : chromatogramme HPLC de l'extrait CCV.

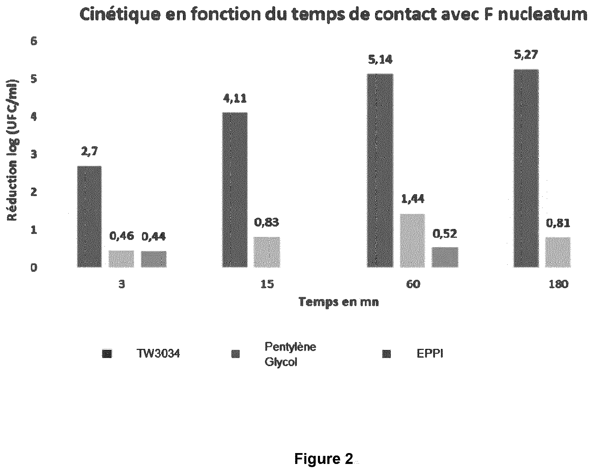

**Figure 2** : réduction du nombre de Log (d'UFC/mL) par rapport au T0, après contact avec les échantillons témoins Eau purifiée pour injectable (EPPI) et pentylène glycol ou de l'échantillon extrait CCV (TW3034) pendant 3 min (T3), 15 min (T15), 60 min (T60) et 180 min (T180).

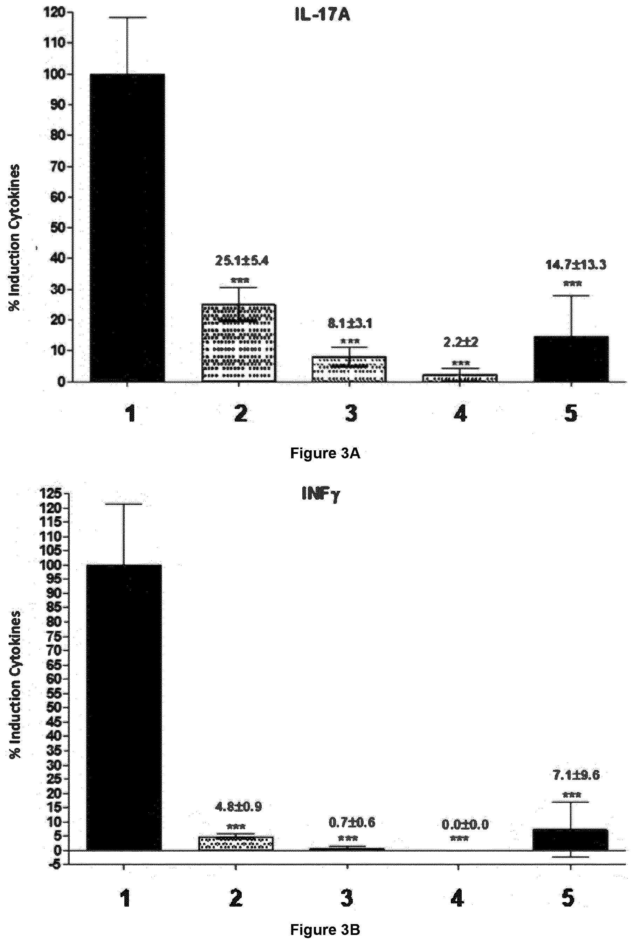

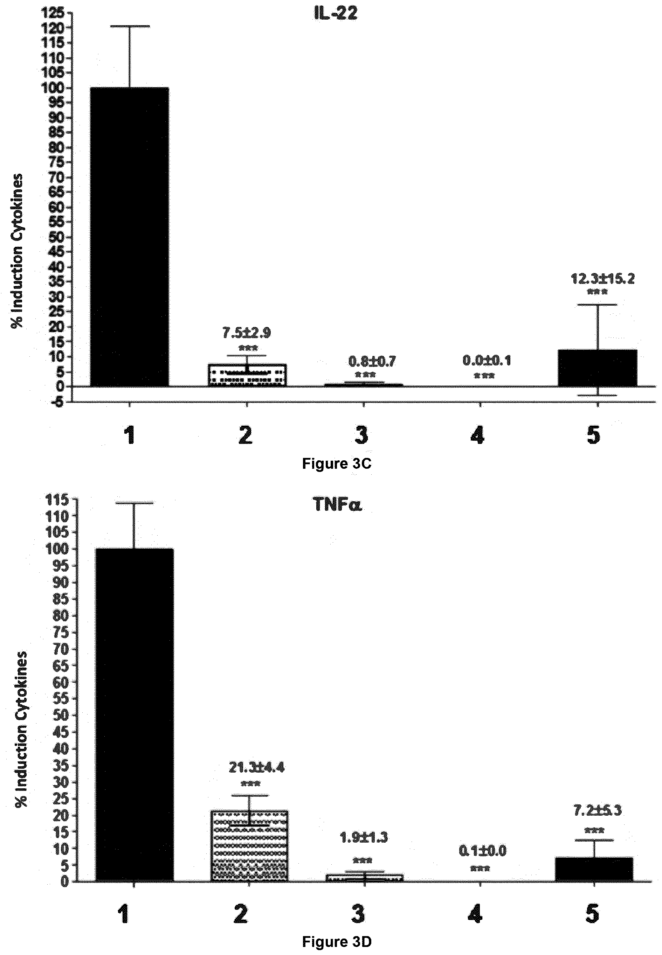

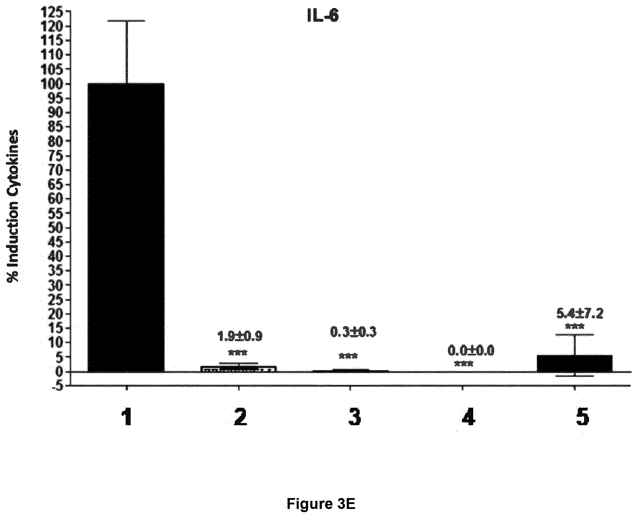

**Figures 3A-3E** : Pourcentage d'induction de la synthèse d'IL-17A (Fig 3A), de IFN-gamma (Fig 3B), d'IL-22 (Fig 3C) de TNF-alpha (Fig 3D) et d'IL-6 (Fig 3E) par des lymphocytes T CD4+ après incubation avec les échantillons d'extrait CCV titré en concentration en Célastrol : **2** (9 ng/mL), **3** (30 ng/mL), **4** (90 ng/mL) et **5** (contrôle positif Dexa 2 µM) par rapport au témoin négatif incubé avec l'échantillon **1** (contrôle négatif) suivie d'une stimulation avec des anticorps anti-CD3 et anti-CD28 (*** valeur p<0,001).

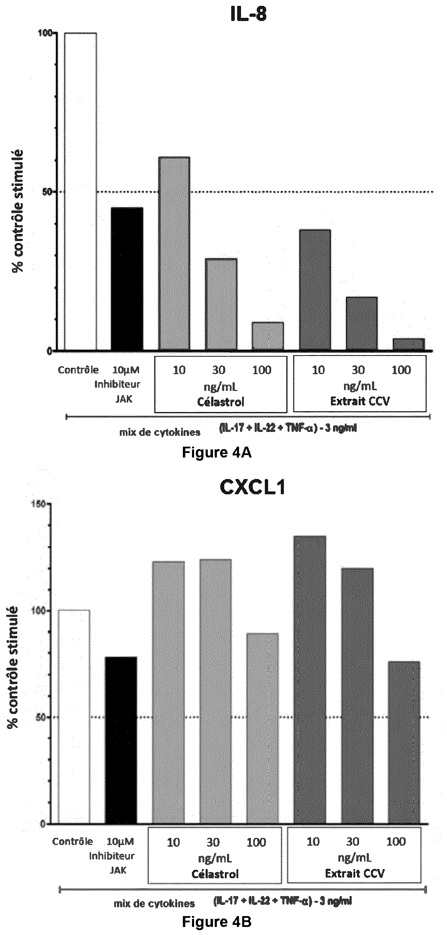

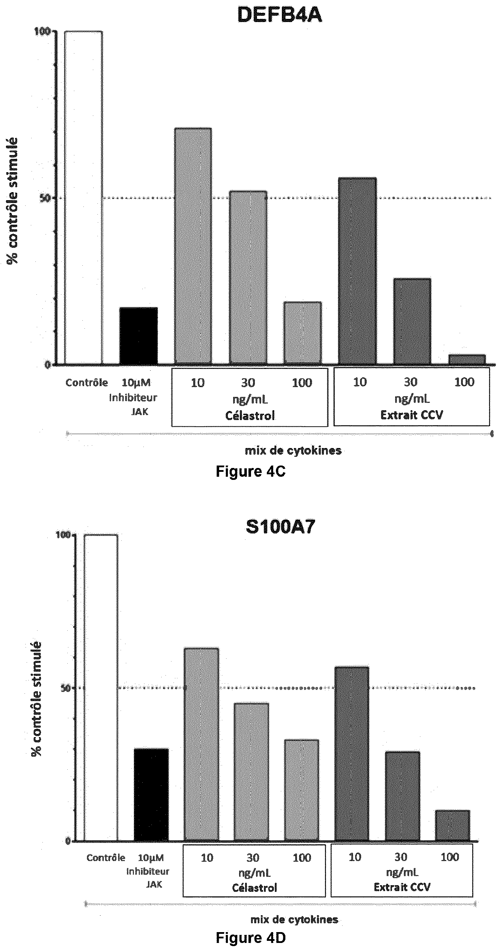

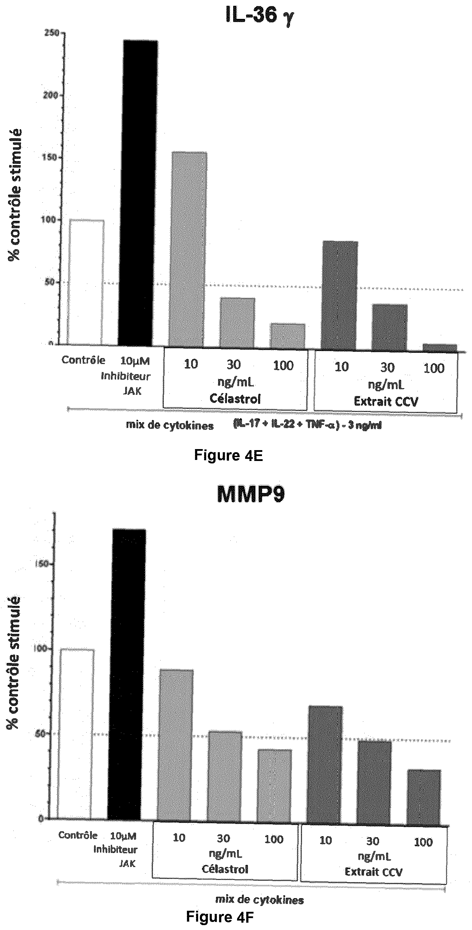

**Figures 4A-4F** : Inhibition, mesurée par RT-qPCR, de l'expression des gènes IL-8 (Fig. 4A), CXCL1 (Fig. 4B), DEFB4A (Fig. 4C), S100A7 (Fig. 4D), IL-36 gamma (Fig. 4E) et MMP-9 (Fig. 4F) par des kératinocytes humains oraux en conditions inflammatoires induites en présence d'un inhibiteur JAK (Témoin positif), d'échantillons « extrait CCV » (à 10, 30 ou 100 ng/mL) ou d'échantillons « Célastrol » (à 10, 30 et 100 ng/mL).

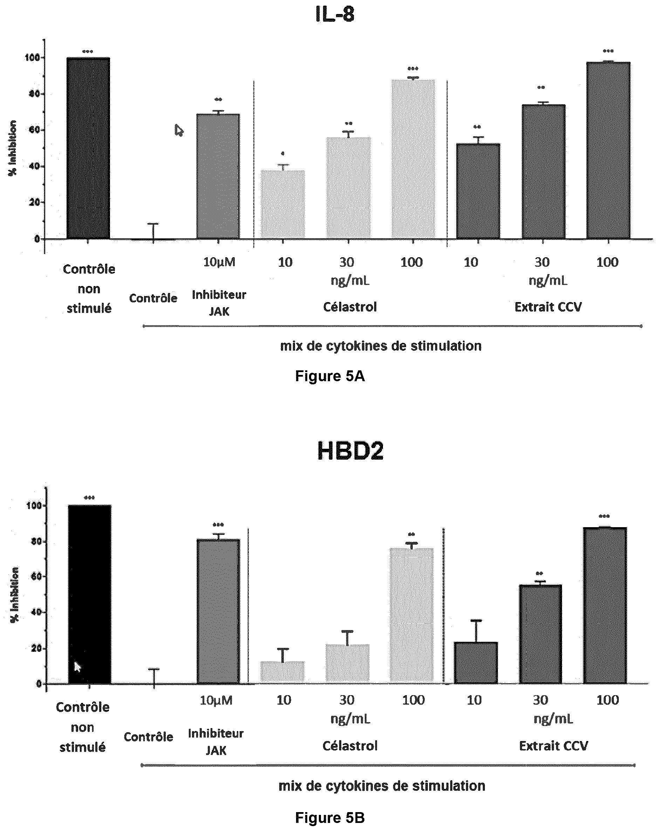

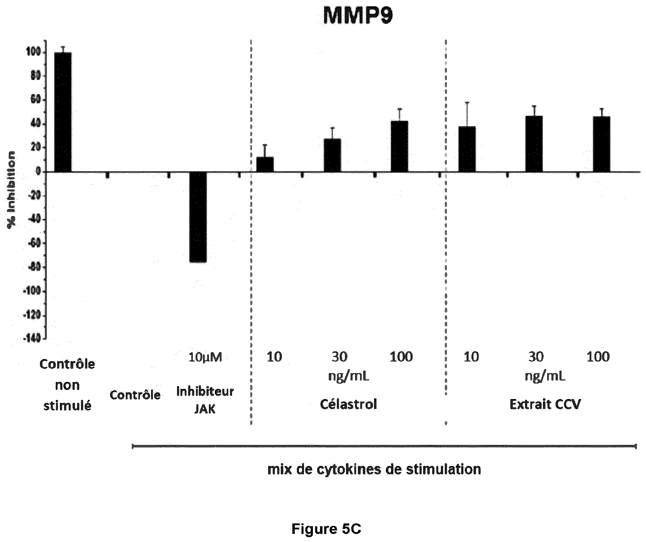

**Figures 5A-5C** : Inhibition, mesurée par ELISA, d'IL-8 (Fig. 5A), HBD2 (Fig. 5B), et MMP-9 (Fig. 5C) par des kératinocytes humains oraux en conditions inflammatoires induites en présence d'un inhibiteur JAK (Témoin positif), d'échantillons « extrait CCV » (à 10, 30 ou 100 ng/mL) ou d'échantillons « Célastrol » (à 10, 30 et 100 ng/mL).

### EXEMPLES :

### EXEMPLE 1 : Obtention de l'extrait de culture de cellules végétales CCV de Tripterygium wilfordii enrichi en triterpènes pentacycliques

On réalise une culture dans un réacteur de type Wave (volume 5 L) Sartorius Stedim Biotech (Germany). On innocule le réacteur avec une suspension de cellules de T. *wilfordii* issus d'un erlen (composition milieu, paramètres cités plus haut). Sous agitation en continu, après avoir atteint le maximum en biomasse environ 17 jours après, on procède à l'élicitation avec le cocktail éliciteur (MejA + Chitine). On arrête la culture après 15 jours d'élicitation. La majorité de la biomasse est récupérée par filtration de la suspension cellulaire avec un filtre nylon (20 - 50 µM). A partir de 5 L de suspension, on récupère environ 1 925 g de biomasse. Cette biomasse est extraite avec de l'acétate d'éthyle (ou encore de l'acétate d'isopropyle) en proportion 2 : 1 (Volume : Poids) par rapport au poids de biomasse (ici 3 850 mL de solvant pour 1 925 g de biomasse). Le mélange biomasse/solvant est alors soumis à une extraction physique, par sonication ou par broyage. La phase organique est alors récupérée après macération sous agitation. L'ajout du solvant (suivi d'une macération sous agitation et de la récupération de la phase organique) est répété 2 fois. On peut concentrer le solvant sous vide, puis solubiliser ensuite dans du pentylène glycol par exemple, et finir l'étape en tirant sous vide afin d'éliminer le solvant organique résiduel. On obtient alors une solution nommée « extrait CCV » contenant les triterpènes pentacycliques suivants : Célastrol (pic majeur), Tingénine A et Tingénine B (voir Fig.1).

Conditions chromatographie HPLC : appareil de chromatographie liquide Alliance (Waters 2695 version 2.03) ; Colonne Sunfire C18, 100Å, 5 µm (4.6 mm X 150 mm). Gradient solvants : Eau/Acétonitrile. Débit: 3 ml/min. Détection des triterpènes à λ 460 nm. Cet extrait, utilisé pour les activités pharmacologiques et anti-bactérienne est titré en concentration en Célastrol. On peut purifier le Célastrol, le Tingénine A et le Tingénine B par collection après séparation par HPLC. Les composés purifiés sont resuspendus dans le DMSO puis dilués dans les tampons adéquats pour chaque test.

### EXEMPLE 2 : Activité antimicrobienne vis-à-vis de Staphylococcus aureus de l'Extrait CCV, et des molécules purifiées de Célastrol, Tingénine A et Tingénine B.

### Matériel et Méthode :

### 1. Obtention des échantillons à tester

- L'échantillon « Extrait CCV » obtenu tel que dans l'exemple 1 est titré à 150 µg/ml en Célastrol, 40 µg/ml en Tingénine A et 20 µg/ml en Tingénine B, dissouts dans du pentylène glycol 100 %. Pour les besoins du dosage, il est dilué à 2 % dans de l'eau pour préparation injectable (PPI).
- L'échantillon « Tingénine A » est titré à 150 µg/ml dans du DMSO 10 %.
- L'échantillon « Tingénine B » est titré à 150 µg/ml du DMSO 10 %.
- L'échantillon « Célastrol » est titré à 150 µ/ml dans du DMSO 10 %.

Les échantillons témoins « DMSO » et « pentylène glycol » sont dilués respectivement à 10 % et à 2 % dans de l'eau PPI.

### 2. Souches bactériennes et entretien

Les souches bactériennes utilisées sont :
- *Staphylococcus aureus* CIP4.83 entretenue sur milieu gelosé Trypticase-soja à 36 +/- 1 °C.
- *Fusobacterium nucleatum* CIP101130T entretenue sur milieu gélosé Columbia + 5 % de sang de mouton à 36+/-1 °C en anaérobiose

### 3. Détermination des CMI et CMB

La détermination de la Concentration Minimale Inhibitrice (CMI) est réalisée par micro-méthode en milieu liquide. 100 µL de milieu de culture Muller Hinton sont déposés dans chaque puits d'une microplaque de 96 puits stérile. 100 µL de l'échantillon à tester sont déposés dans la colonne 1 de la microplaque (deux puits par extrait à tester). Des dilutions de raison 2 sont réalisées de la façon suivante : 100 µL sont prélevés dans les puits de la colonne 1 puis ajouté et mélangés aux puits de la colonne 2. Cette opération est répétée jusqu'à la colonne 10. Les 100 µL prélevés dans la colonne 10 sont éliminés. La colonne 11 est utilisée comme témoin positif (milieu de culture + bactérie à tester). La colonne 12 est utilisée comme témoin négatif (milieu de culture seul). La microplaque est ensemencée (hors témoin négatif de croissance). Après une incubation de 16 à 24 h l'observation de la croissance à l'œil nu permet de déterminer une CMI (la plus faible concentration en extrait qui inhibe la croissance).

La détermination de la Concentration Minimale Bactéricide (CMB) est réalisée par repiquage des microplaques CMI sur un milieu Muller Hinton gélosé. Après incubation, la CMB correspond à la plus faible concentration de l'extrait testé pour laquelle on observe une absence de croissance.

Dans les tableaux 1 et 2 présentant les résultats comparatifs des différents échantillons testés sont indiqués :
1. Pour l'extrait CCV, les CMI/CMB sont des concentrations équivalentes en célastrol. En effet, cet extrait est une solution qui contient majoritairement du Célastrol (Célastrol > 50 %, Tingénine A > 5 % et Tingénine B > 5 %). L'extrait est dissout dans du Pentylène Glycol (PG), on a le % (P/P) correspondant à la dilution réelle au point de CMI ou de CMB, et on en déduit la concentration équivalente en Célastrol grâce au titre (150 µg/ml) quantifié par HPLC.
2. Pour les échantillons de Célastrol, de Tingénine A et de Tingénine B purifiés et dissouts dans le DMSO, le % mentionné correspond à la dilution réelle au point de CMI ou de CMB.
3. Pour les solvants de contrôle DMSO et Pentylène Glycol (PG) testés en parallèle, le % correspond à la dilution réelle au point de CMI ou de CMB.

### Résultats :

### i. S. aureus

Les valeurs de CMI et CMB obtenues sur S. *aureus* sont reprises dans le tableau 1 ci-dessous :

**Tableau 1 : valeurs de CMI et CMB obtenues sur S. aureus pour les échantillons**

| **Echantillon** | **CMI** | **CMB** |
|---|---|---|
| Célastrol | 0,29 µg/ml (DMSO dilution à 0,01 %) | 0,59 µg/ml (DMSO dilution à 0,04 %) |
| Tingénine A | 1,17 µg/ml (DMSO dilution à 0,08 %) | 2,34 µg/ml (DMSO dilution à 0,08 %) |
| Tingénine B | 0,59 µg/ml (DMSO dilution à 0,04 %) | 1,17 µg/ml (DMSO dilution à 0,08 %) |
| DMSO | Dilution supérieure à 5 % | Dilution supérieure à 5 % |
| Extrait CCV | 0,19 µg/ml (concentration équivalente en célastrol) (0,125 % solubilisé dans PG) | 0,4 µg/ml (concentration équivalente en célastrol) (0,25 % solubilisé dans PG) |
| Pentylène glycol (PG) | Dilution supérieure à 1 % | Dilution supérieure à 1 % |

On constate que le Célastrol, l'Extrait CCV, la Tingénine A et la Tingénine B ont une activité antibactérienne significative contre *S*. *aureus,* indépendante de celle du DMSO ou du pentylène glycol. La meilleure activité antibactérienne est obtenue avec l'extrait CCV.

Par ailleurs, la Tingénine B a une activité anti *S*. *aureus* 2 à 4 fois supérieure à celle de la Tingénine A.

### ii. F. nucleatum

Les valeurs de CMI et CMB obtenues sur *F. nucleatum* sont reprises dans le tableau 2 ci-dessous :

**Tableau 2 : valeurs de CMI et CMB obtenues sur F. nucleatum pour les échantillons**

| **Echantillon** | **CMI** | **CMB** |
|---|---|---|
| Extrait CCV | 0,03 % dans le PG (équivalant à une concentration de 0,045 µg/ml en célastrol | 0,03 % dans le PG (équivalant à une concentration de 0,045 µg/ml en célastrol |
| Pentylène glycol (PG) | dilution supérieure 1 % | dilution supérieure 1 % |

L'extrait CCV, qui comprend les trois triterpènes dans le Pentylène glycol a une forte activité bactéricide sur *F. nucleatum.* Le PG seul a une activité CMI seulement à la dilution supérieure de 1 %. Le PG n'est qu'à 0,03 % dans l'extrait CCV, donc on ne peut pas attribuer l'activité CMI à lui seul.

### EXEMPLE 3 : Cinétique bactéricide de l'extrait CCV sur F. nucleatum.

### Matériel et Méthode :

### 1. Echantillons testés

L'échantillon « Extrait CCV » est obtenu tel que décrit dans l'exemple 1 et contient du Célastrol, de la Tingénine A et de la Tingénine B dans du Pentylène Glycol. Il est testé à concentration équivalente en Célastrol de 1,2 µg/mL, ce qui correspond à 0,8% de Pentylène Glycol au final.

### 2. Souche bactérienne et entretien

La souche bactérienne utilisée est la souche de *Fusobacterium nucleatum* CIP101130T entretenue sur milieu gélosé Schaedler et le dénombrement des UFC est réalisée par inclusion en gélose Schaedler à 36 +/-1 °C en anaérobiose.

### 3. Cinétique

La cinétique de croissance bactérienne est réalisée de la manière suivante
- 1 ml de suspension bactérienne est ajouté à 9 ml d'échantillon. 1 ml est prélevé directement après homogénéisation et mis en contact avec 9 ml de solution de neutralisant (selon la norme européenne NF EN 14885 : neutralisation par dilution pendant 5 minutes dans un mélange Polysorbate 80 10 %, Saponine 2 %, Lécitine 2 %, Thiosulfate de sodium 0,5 % qsp bouillon trypticase soja). Cet échantillon correspond au T0.
- La suspension restante est placée à 32 °C. Des échantillons de 1 ml correspondant au T3, T15, T60 et T180 sont prélevés respectivement à 3 minutes, 15 minutes, 60 minutes et 180 minutes et neutralisés comme précédemment.
- 5 dilutions de raison 10 sont réalisées, en eau PPI à partir des échantillons neutralisés. Les dilutions de 10⁻³ à 10⁻⁵ sont dénombrées sur boîtes de gélose de Schaedler (ensemencement par inclusion en duplicata).
- Le nombre d'UFC est dénombré pour chacune des dilutions. Le nombre d'UFC est ramené à 1 ml d'extrait à analyser.

Les résultats obtenus sont exprimés en réduction du nombre de Log (UFC/ml) par rapport au nombre de bactéries présentes au temps T0. Les résultats de chaque extrait sont résumés dans le tableau 3 et représentés Fig. 2

**Tableau 3 : réduction du nombre de Log d'UFC/ml par rapport au T0 après contact avec les échantillons**

| **Echantillon/temps « min »** | **T3mn** | **T15mn** | **T60mn** | **T180mn** |
|---|---|---|---|---|
| Eau PPI | 0,44 | ND | 0,52 | ND |
| Pentylène glycol dilué à 0,8% | 0,46 | 0,83 | 1,44 | 0,81 |
| Extrait CCV (concentration équivalente en célastrol de 1,2 µg/ml) | 2,7 | 4,11 | 5,14 | 5,27 |

Ces résultats démontrent une forte activité bactéricide de l'extrait CCV. Dès 3 minutes de contact, une réduction de 2,7 log de la charge bactérienne est mise en évidence. L'allongement du temps de contact permet d'obtenir après 4h, une réduction de 5,2 log. A l'inverse, le contrôle PG seul, dilué à 0,8 %, concentration identique à celle de l'extrait CCV, n'a pas d'effet sur la charge bactérienne.

### EXEMPLE 4 : Activité anti-TH17 de l'Extrait CCV :

### Matériel et Méthode :

### 1. Obtention des échantillons à tester

L'échantillon « Extrait CCV » est obtenu tel que dans l'exemple 1 et est dilué dans 0,4 % DMSO afin d'obtenir les différentes concentrations finales suivantes après ajout dans les puits :
Extrait **2** (titré en Célastrol à 9 ng/ml) ;
Extrait **3** (titré en Célastrol à 30 ng/ml) ; et
Extrait **4** (titré en Célastrol à 90 ng/ml).

Les échantillons contrôles sont les suivants :
Contrôle négatif **1** (solution DMSO à 0,4 %) ; et
Contrôle positif **5 :** dexaméthasone (Dexa) à 2 µM (concentration finale dans le puits).

### 2. Isolement de cellules CD4+ et Analyse des cytokines secrétées

Les cellules T humaines ont été isolées à partir de cellules mononuclées de sang de deux donneurs sur Ficoll Paque plus (selon le protocole du fabricant). Les cellules CD4+ sont isolées par tri positif (Kit Miltenyi Biotec (CD4) et colonne LS) et remises en suspension dans du milieu de culture RPMI (Sigma Aldrich : L-Glutamine et sérum foetal bovin à 10 %) additionné de 100 pg/mL de streptomycine et 100 U/mL de pénicilline.

Les Lymphocytes en suspension sont répartis dans des puits de microplaques. Les échantillons extraits CCV 2, 3 et 4 à tester sont ensuite ajoutés dans chacun des puits pour atteindre les concentrations en Célastrol de 9, 30 et 90 ng/ml respectivement. Les plaques sont maintenues à température ambiante pendant deux heures puis les lymphocytes sont activés pour induire une réponse de type TH17 : 20 heures à 37 °C en présence de 300 ng/ml d'anticorps anti-CD3 et 400 ng/mL d'anticorps anti-CD28.

La quantité de cytokine IL17A, IFN gamma, IL22, TNF alpha et IL6 sécrétée dans le surnageant de culture des lymphocytes est ensuite mesurée par la méthode Multiplex Immunoassays (tel que notamment décrit dans Jager et al. Clin. Diagn. Lab. Immunol. 2003, 10(1), 133-139).

### Résultats :

Les résultats obtenus sont présentés en Figures 3A, 3B, 3C et 3E en pourcentage d'induction par rapport au témoin négatif. On constate que :
- le contrôle positif **5** (le dexaméthasone à 2 µM) inhibe la surexpression de toutes les cytokines : IL-17A, IFN gamma, IL-22, TNF alpha et IL-6 ; et
- les extraits CCV (**2**, **3** et **4**) inhibent fortement la surexpression des cytokines IL-17A, IFN gamma, IL-22, TNF alpha et IL-6. L'inhibition de la surexpression d'IL-17A et TNF alpha est dose-dépendante. Dès la plus faible dose, **2** titrée à 9 ng/ml en Célastrol, on constate une inhibition de la surexpression de l'IL-17A de 75 %, une inhibition de la surexpression du TNF alpha de 80 % et une inhibition presque totale de la surexpression de l'INF-gamma, de l'IL-22 et de l'IL-6.

Ceci démontre que l'Extrait CCV a une activité anti-Th17 très forte, comparable sinon supérieure à la dexaméthasone (2 µM).

### EXEMPLE 5 : Activité de l'Extrait CCV et du célastrol sur les kératinocytes oraux :

### Matériel et Méthode :

### 1. Echantillons testés

- L'échantillon « Extrait CCV » est obtenu tel que décrit dans l'exemple 1 et est dilué dans du DMSO 0,4% afin d'obtenir une concentration en célastrol de 137 µg/mL ; trois doses sont testées, à savoir 10, 30 et 100 ng/mL.
- L'échantillon « Célastrol » 150 µg/mL dans du DMSO 0,4 % ; trois doses sont testées à savoir 10, 30 et 100 ng/mL.

Les échantillons contrôles sont les suivants :
- L'échantillon témoin négatif « DMSO » (DMSO à 0,4%) ; et
- L'échantillon témoin positif « Inhibiteur JAK » comprenant 10 µM d'inhibiteur JAK (JAK Inhibitor I - CAS 457081-03-7 - Calbiochem).

### 2. Lignée cellulaire

La lignée cellulaire utilisée est une lignée de kératinocytes humains oraux (CliniSciences ref.2610-SC). Cette lignée est cultivée à 37 °C, sous atmosphère 5 % de CO₂ dans un milieu adapté (Oral Keratinocyte medium OKM supplémenté avec de la pénicilline 100 U/mL et de la streptomycine 100 µg/mL).

### 3. Stimulation des kératinocytes

Les kératinocytes humains oraux sont ensemencés dans des puits de plaques à 24 puits (75 000 cellules/puits) et cultivés pendant 24h. Le milieu de culture est alors remplacé par du milieu frais contenant les échantillons à tester, et les cellules sont à nouveau incubées pendant 24h. L'opération est renouvelée à nouveau en présence d'un mix de cytokines stimulatrice des kératinocytes : IL-17, IL-22 et TNF-alpha à 3 ng/ml chacune. Le temps de culture est de 24h pour les expériences de RT-pPCR et de 48 pour les expériences d'ELISA. Les cellules sont ensuite lavées dans une solution de PBS et congelées immédiatement à - 80 °C.

Les expériences ont été réalisées en triplicats.

### 4. RT-qPCR.

L'expression des marqueurs IL-8, CXCL1, DEBF4A, S100A7, IL-36 gamma et MMP9, par les kératinocytes est déterminée par RT-qPCR comme décrit dans la littérature avec des kits de Reverse transcriptase (Roche) et de PCR quantitative. Un témoin positif de PCR (GAPDH), exprimé constitutivement, est réalisé en parallèle afin de normaliser les résultats obtenus.

### 5. ELISA

La quantité d'IL8, hBD-2 (DEBF4A) et de MMP-9 sécrétée par les kératinocytes dans le milieu de culture après 48h de stimulation est mesurée par ELISA.

### Résultats :

### A. Les résultats de RT-qPCR obtenus sont représentés Fig. 4A à 4F :

Le traitement des kératinocytes humains oraux avec le mix de cytokines de stimulation induit une augmentation claire et significative de l'expression des marqueurs IL-8 (Fig. 4A), CXCL1 (Fig. 4B) DEBF4 (Fig. 4C), S100A7 (Fig. 4D), IL-36 gamma (Fig. 4E) et MMP9 (Fig. 4F).

L'échantillon témoin positif (inhibiteur JAK) induit une diminution de l'expression d'IL8, de DEFB4A et de S100A7 par rapport à l'échantillon témoin négatif. En revanche, il n'inhibe ni l'IL-36 gamma ni la MMP9 à cette dose. A noter que l'inhibiteur JAK I utilisé est connu dans la littérature pour potentialiser l'expression de MMP9 par une autre voie mécanistique (Kothari et al. J. Immunol. 2014, 192, 349).

Les échantillons extraits CCV et les échantillons Célastrol induisent une inhibition claire, significative et dose dépendante de l'expression des marqueurs IL-8, DEFBA4, S100A7, IL-36 gamma et MMP9. Ces échantillons ont donc une activité anti-inflammatoire sur les kératinocytes humais oraux, l'extrait CCV donnant de meilleurs résultats que le Célastrol seul. Et de manière surprenante, l'extrait CCV et le Célastrol inhibent l'expression de MMP9 par les kératinocytes oraux.

### B. Les résultats obtenus par ELISA sont représentés Figures 5A, 5B et 5C :

En absence de stimulation, la sécrétion d'IL-8 ou de MMP-9 par les kératinocytes oraux est faible (126 pg/mL et 201 pg/mL respectivement), la sécrétion de β-défensine-2 (HBD2) est non détectable.

Le traitement avec le mix de cytokines de stimulation induit une augmentation claire et significative de l'expression d'IL-8 (1 630 pg/mL) (Fig. 5A), de MMP-9 (531 pg/mL) (Fig. 5C) et de β-défensine-2 (253 pg/ml) (Fig. 5B).

En comparaison, l'échantillon témoin positif (inhibiteur JAK) induit une inhibition de la sécrétion d'IL-8 (69 % d'inhibition), de β-défensine-2 (82 % d'inhibition) et une augmentation de la sécrétion de MMP-9 (60 % d'augmentation), ce qui est en corrélation avec les résultats d'expression génique RT-PCR.

Les échantillons extraits CCV induisent une inhibition claire, significative et dose dépendante de l'expression des marqueurs IL-8 (de 38 % à 88 % selon la concentration de l'extrait), β-défensine-2 (24 à 88 % selon la concentration de l'extrait) et MMP9 (de 10 à 50 % selon la concentration).

Les échantillons Célastrol induisent une inhibition claire, significative et dose dépendante de l'expression des marqueurs IL-8 (de 53 % à 98 % selon la concentration en Célastrol), β-défensine-2 (13 à 76 % selon la concentration en célastrol) et MMP9 (d'environ 40 % à la concentration de 30 ng/ml).

Ces résultats démontrent encore une fois que l'extrait CCV a un fort pouvoir d'inhibiteur de réponses Th17, tout comme le Célastrol, l'extrait CCV ayant un pouvoir inhibiteur supérieur à celui du Célastrol seul, à concentration équivalente.

## Revendications

1. Composition comprenant au moins un triterpène pentacyclique ou l'un de ses sels pharmaceutiquement acceptables pour son utilisation dans le traitement et/ou la prévention d'une pathologie bucco-dentaire, dans laquelle le au moins un triterpène pentacyclique est un mélange de Célastrol, de Tingénine A et de Tingénine B.

2. Composition pour son utilisation selon la revendication 1, dans laquelle les triterpènes pentacycliques ou leurs sels pharmaceutiquement acceptables sont présents au sein d'un extrait d'une plante de la famille des Celastraceae.

3. Composition pour son utilisation selon la revendication 2, dans laquelle la plante de la famille des Celastraceae est choisie parmi *Tripterygium wilfordii, Tripterygium hypoglaucum* et *Celastrus orbiculatus.*

4. Composition pour son utilisation selon la revendication 2 ou 3, dans laquelle l'extrait est un extrait brut enrichi obtenu par le procédé suivant :
(i) une phase de prolifération de cellules d'une plante de la famille des Celastraceae dans un milieu de prolifération,
(ii) une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) la préparation d'un extrait brut enrichi en triterpènes pentacycliques à partir de la culture cellulaire obtenue à l'étape (ii).

5. Composition pour son utilisation selon la revendication 4, comprenant entre 0,01 et 2 % d'extrait brut enrichi en poids par rapport au poids total de la composition.

6. Composition pour son utilisation selon la revendication 4 ou 5, dans laquelle l'extrait brut enrichi comprend entre 90 et 100 % de triterpènes pentacycliques en poids par rapport au poids total de l'extrait brut enrichi.

7. Composition pour son utilisation selon l'une quelconque des revendications 4 à 6, dans laquelle l'extrait brut enrichi comprend :
- entre 1 et 20%, notamment entre 5 et 15 %, notamment entre 8 et 12 % de Tingénine A en poids par rapport au poids total de l'extrait,
- entre 1 et 20 %, notamment entre 5 et 15 %, notamment entre 8 et 12 % de Tingénine B en poids par rapport au poids total de l'extrait, et
- au moins 60 %, notamment au moins 80% de Célastrol en poids par rapport au poids total de l'extrait.

8. Composition pour son utilisation selon l'une quelconque des revendications 1 à 7, sous forme d'un bain de bouche, d'un gel ou d'une pâte dentifrice, d'un gel gingival, d'une gomme à mâcher, d'une pastille à sucer, d'un fil dentaire, d'une capsule extrudée, de brins pour brosses à dents, d'un spray, d'un pansement adhésif, d'une lingette imprégnée, d'une pâte adhésive pour dentiers et prothèses ou d'une pâte prophylactique de polissage, de préférence sous forme d'une pâte dentifrice ou d'un bain de bouche.

9. Composition pour son utilisation selon l'une quelconque des revendications 1 à 8, comprenant entre 0,1 et 2 % en poids de pentylène glycol par rapport au poids total de la composition.

10. Composition pour son utilisation selon l'une quelconque des revendications 1 à 9, dans laquelle la pathologie bucco-dentaire est liée à la présence de bactéries pathogènes, notamment *S*. *aureus* et/ou *F. nucleatum.*

11. Composition pour son utilisation selon l'une quelconque des revendications 1 à 10, dans laquelle la pathologie bucco-dentaire est médiée par les cytokines et les chémiokines de type Th17.

12. Composition pour son utilisation selon l'une quelconque des revendications 1 à 11, dans laquelle la pathologie bucco-dentaire est la parodontite, la gingivite, la péri-mucosite, la péri-implantite ou le lichen plan buccal.

13. Composition pour son utilisation selon la revendication 12, dans laquelle la pathologie bucco-dentaire est la parodontite ou la gingivite.

## Patentansprüche

1. Zusammensetzung, umfassend mindestens ein pentazyklisches Triterpen oder eins seiner pharmazeutisch akzeptablen Salze für ihre Verwendung zur Behandlung und/oder zur Vorbeugung einer oralen Erkrankung, wobei das mindestens eine pentazyklisches Triterpen ein Gemisch aus Celastrol, Tingenin A und Tingenine B ist.

2. Zusammensetzung für ihre Verwendung nach Anspruch 1, wobei die pentazyklischen Triterpene oder ihre pharmazeutisch akzeptablen Salze in einem Extrakt einer Pflanze aus der Familie der Celastraceae vorhanden sind.

3. Zusammensetzung für ihre Verwendung nach Anspruch 2, wobei die Pflanze aus der Familie der Celastraceae aus *Tripterygium wilfordii, Tripterygium hypoglaucum* und *Celastrus orbiculatus* ausgewählt ist.

4. Zusammensetzung für ihre Verwendung nach Anspruch 2 oder 3, wobei der Extrakt ein angereicherter Rohextrakt ist, erhalten durch das folgende Verfahren:
(i) eine Phase der Vermehrung von Zellen einer Pflanze aus der Familie der Celastraceae in einem Vermehrungsmedium,
(ii) eine Phase der Elicitation durch Hinzufügen eines Elicitationscocktails zu der in Schritt (i) erhaltenen Zellkultur, wobei der Elicitationscocktail mindestens einen Elicitor vom Typ Monocarbonverbindung und mindestens einen biotischen Elicitor umfasst, und
(iii) die Zubereitung eines mit pentazyklischen Triterpenen angereicherten Rohextrakts aus der in Schritt (ii) erhaltenen Zellkultur.

5. Zusammensetzung für ihre Verwendung nach Anspruch 4, umfassend zwischen 0,01 und 2 Gew.-% angereicherten Rohextrakt im Verhältnis zum Gesamtgewicht der Zusammensetzung.

6. Zusammensetzung für ihre Verwendung nach Anspruch 4 oder 5, wobei der angereicherte Rohextrakt zwischen 90 und 100 Gew.-% pentazyklische Triterpene im Verhältnis zum Gesamtgewicht des angereicherten Rohextrakts umfasst.

7. Zusammensetzung für ihre Verwendung nach einem der Ansprüche 4 bis 6, wobei der angereicherte Rohextrakt umfasst:
- zwischen 1 und 20, insbesondere zwischen 5 und 15, insbesondere zwischen 8 und 12 Gew.-% Tingenin A im Verhältnis zum Gesamtgewicht des Extrakts,
- zwischen 1 und 20, insbesondere zwischen 5 und 15, insbesondere zwischen 8 und 12 Gew.-% Tingenin B im Verhältnis zum Gesamtgewicht des Extrakts, und
- mindestens 60, insbesondere mindestens 80 Gew.-% Celastrol im Verhältnis zum Gesamtgewicht des Extrakts.

8. Zusammensetzung für ihre Verwendung nach einem der Ansprüche 1 bis 7 in Form einer Mundspülung, eines Gels oder einer Zahncreme, eines Gingivalgels, eines Kaugummis, einer Lutschpastille, einer Zahnseide, einer extrudierten Kapsel, von Borsten für Zahnbürsten, eines Sprays, eines Pflasters, eines imprägnierten Tuchs, einer Haftcreme für Zahnersatz und Prothesen oder einer prophylaktischen Polierpaste, vorzugsweise in Form einer Zahncreme oder einer Mundspülung.

9. Zusammensetzung für ihre Verwendung nach einem der Ansprüche 1 bis 8, umfassend zwischen 0,1 und 2 Gew.% Pentylenglycol im Verhältnis zum Gesamtgewicht der Zusammensetzung.

10. Zusammensetzung für ihre Verwendung nach einem der Ansprüche 1 bis 9, wobei die orale Erkrankung mit dem Vorhandensein krankmachender Bakterien, insbesondere von *S. aureus* und/oder *F. nucleatum,* verbunden ist

11. Zusammensetzung für ihre Verwendung nach einem der Ansprüche 1 bis 10, wobei die orale Erkrankung von den Cytokinen und den Chemiokinen vom Typ Th17 vermittelt wird.

12. Zusammensetzung für ihre Verwendung nach einem der Ansprüche 1 bis 11, wobei die orale Erkrankung die Parodontitis, die Gingivitis, die Peri-Mucositis, die Peri-Implantitis oder der orale Lichen planus ist.

13. Zusammensetzung für ihre Verwendung nach Anspruch 12, wobei die orale Erkrankung die Parodontitis oder die Gingivitis ist.

## Claims

1. A composition comprising at least one pentacyclic triterpene or one of the pharmaceutically acceptable salts thereof for use in the treatment and/or prevention of an oral-dental pathology, wherein the at least one pentacyclic triterpene is a mix of celastrol, tingenin A, and tingenin B.

2. The composition for use according to claim 1, wherein the pentacyclic triterpenes or the pharmaceutically acceptable salts thereof are present in an extract of a plant of the *Celastraceae* family.

3. The composition for use according to claim 2, wherein the plant of the *Celastraceae* family is selected from *Tripterygium wilfordii, Tripterygium hypoglaucum* and *Celastrus orbiculatus.*

4. The composition for use according to claim 2 or 3, wherein the extract is an enriched crude extract obtained by the following process:
(i) a phase of proliferation of cells of a plant of the *Celastraceae* family in a proliferation medium,
(ii) a phase of elicitation by adding an elicitation cocktail to the cell culture obtained in step (i), said elicitation cocktail containing at least one monocarboxylic compound-type elicitor and at least one biotic elicitor, and
(iii) preparation of a crude extract enriched in pentacyclic triterpenes from the cell culture obtained in step (ii).

5. The composition for use according to claim 4, comprising between 0.01 and 2 % enriched crude extract by weight based on the total weight of the composition.

6. The composition for use according to claim 4 or 5, wherein the enriched crude extract comprises between 90 and 100 % pentacyclic triterpenes by weight based on the total weight of the enriched crude extract.

7. The composition for use according to any one of claims 4 to 6, wherein the enriched crude extract comprises:
- between 1 and 20 %, notably between 5 and 15 %, notably between 8 and 12 % tingenin A by weight based on the total weight of the extract,
- between 1 and 20 %, notably between 5 and 15 %, notably between 8 and 12 % tingenin B by weight based on the total weight of the extract, and
- at least 60 %, notably at least 80 % celastrol by weight based on the total weight of the extract.

8. The composition for use according to any one of claims 1 to 7, in the form of a mouthwash, a toothpaste or gel, a gingival gel, a chewing gum, a lozenge, a dental floss, an extruded capsule, toothbrush bristles, a spray, an adhesive dressing, an impregnated wipe, an adhesive paste for dentures and prostheses, or a prophylactic polishing paste, preferably in the form of a toothpaste or a mouthwash.

9. The composition for use according to any one of claims 1 to 8, comprising between 0.1 and 2 % by weight of pentylene glycol based on the total weight of the composition.

10. The composition for use according to any one of claims 1 to 9, wherein the oral-dental pathology is related to the presence of pathogenic bacteria, notably S. *aureus* and/or *F. nucleatum.*

11. The composition for use according to any one of claims 1 to 10, wherein the oral-dental pathology is mediated by Th17-type cytokines and chemokines.

12. The composition for use according to any one of claims 1 to 11, wherein the oral-dental pathology is periodontitis, gingivitis, peri-implant mucositis, peri-implantitis or oral lichen planus.

13. The composition for use according to claim 12, wherein the oral-dental pathology is periodontitis or gingivitis.
